# EUROPÄISCHE PATENTANMELDUNG

(11) **EP 2 289 472 A2**
(43) Veröffentlichungstag der Anmeldung: **02.03.2011**
(21) Anmeldenummer: 10400039.3
(22) Anmeldetag: 28.07.2010
(51) Int. Cl.: A61F 13/04

(54) **Körperverband**

(30) Priorität: 01.08.2009 DE 202009010467 U
(71) Anmelder: Hans-Peter, Zepf, 72280 Dornstetten (DE); Stefan, Köhl, 72280 Dornstetten (DE)
(72) Erfinder: Hans-Peter, Zepf, 72280 Dornstetten (DE); Stefan, Köhl, 72280 Dornstetten (DE)

(57) **Zusammenfassung**

Eine Schicht oder ein Bauteil 2 aus einem saugfähigen und flüssigkeitstransportfähigen Material oder aus einem Material mit geringem Strömungswiderstand für gasförmige Substanzen, angeordnet auf der körperzugewandten Seite eines medizinischen Verbandes 3, erlaubt es, bei angelegtem Verband Flüssigkeiten oder gasförmige Stoffe an Körperpartien 1 unterhalb des Verbandes zu applizieren und dadurch diese Körperpartien ohne Entfernung des Verbandes mit medizinisch oder therapeutisch wirksamen Substanzen zu versorgen.

## Beschreibung

Die vorliegende Erfindung betrifft einen medizinischen Körperverband mit mindestens in Teilzonen mehrschichtigem Aufbau, wobei mindestens eine äußere, vom Körper abgewandte Schicht therapeutisch funktionelle Funktionen wie Stabilisierung Lagerung, Fixierung, Korrektur oder Wundabdeckung übernimmt und eine vollflächig oder zumindest in Teilflächen angeordnete innere, der Körperoberfläche zugewandte Schicht aus saugfähigem und flüssigkeitstransportfähigem Material besteht.

Unter dem Begriff "Körperverband" werden Verbände wie Wundverbände und Stützverbände, aber auch Bandagen, Orthesen oder Schienen verstanden.

Aufgabe der Erfindung ist es, die Versorgung der unter dem Verband liegenden Körperpartien zu ermöglichen, ohne den Verband öffnen oder entfernen zu müssen.
Die Versorgung erfolgt durch Substanzen in flüssiger, insbesondere gelöster Form, auch durch Pasten oder Gels geringer Viskosität. Sie kann aber auch durch gasförmige Substanzen erfolgen oder einfach durch Luft zur Belüftung oder Trocknung der betreffenden Körperpartien. Diese Substanzen können insbesondere medizinische Wirkstoffe sein, die zur Sterilisierung, Wundheilung, Abschwellung, Entzündungshemmung, zur Durchblutungs- oder Stoffwechselförderung oder Hautpflege eingesetzt werden. Die Substanzen können oberflächliche Wirkungen auf die Haut ausüben oder über die Haut aufgenommen im Körper wirksam sein. Die Aufgabe wird erfüllt durch die Merkmale der Ansprüche 1 bis 4 dieser Erfindung.

Es ist insbesondere bei der Wundversorgung gebräuchlich, die Wunde unter dem Wundverband medikamentös zu behandeln. Im Allgemeinen wird eine Dosis des Substanz vor der Abdeckung der Wunde durch den Verband auf die Wunde aufgebracht. Ebenso wird eine medizinisch wirksame Dosis häufig der Abdeckung der Wunde appliziert.
Nachteilig an all diesen Lösungen ist zum einen, dass die Wirkung des Medikaments entsprechend der Vorratsmenge der Substanz zu Behandlungsbeginn groß ist, im Laufe der Zeit aber kontinuierlich abnimmt und zum anderen, dass ein Einfluß auf die Behandlung bei angelegtem Verband nicht mehr möglich ist.
Durch die deutsche Patentschrift DE 60022785 wird deshalb eine Lösung vorgeschlagen, wonach eine Medikamentenlösung durch einen im Verband integrierten Schlauch an den Ort der Behandlung, insbesondere an eine Wunde herangeführt wird. Die Lösung ist recht aufwändig, erfordert mehrere Bauteile, die erstens den Verband versteifen und ihn zweitens beträchtlich aufdicken und sie erlaubt nur eine lokale Anwendung, wobei der Ort der Behandlung nur durch die Anbringung eines neuen Verbandes variiert werden kann. Außerdem ist die Lehre der DE 60022785 nur zur Wundbehandlung anwendbar.

Es ist insbesondere das Ziel der vorliegenden Erfindung, einen Verband zu schaffen, der sowohl eine vollflächige als auch eine lokale Versorgung der vom Verband bedeckten Körperpartien mit flüssigen und gasförmigen Substanzen erlaubt, der einfach und preisgünstig zu realisieren ist, der nur geringe Aufdickungen und unwesentliche Versteifungen zur Folge hat. Der Gegenstand der Erfindung ist nicht nur auf die Anwendung in Wundverbänden beschränkt, sondern schließt jede Heil- und Pflegeversorgung ein, die über die Haut einwirken kann. Der Verband kann unter anderem als Wundverband, als Stützverband, als Bandage oder als Orthese ausgelegt sein.

Die Aufgabe der Erfindung wird erfüllt durch ein Bauteil, das ein saugfähiges und flüssigkeitstransportfähiges Material enthält, das auf der der Körperoberfläche zugewandten Seite eines Körperverbandes angebracht wird oder durch einen mehrschichtigen Verband mit einer der Körperoberfläche zugewandten Schicht aus saugfähigem und flüssigkeitstransportfähigem Material.

Dieses der Körperoberfläche zugewandte Material kann direkt auf der Körperoberfläche aufliegen oder von dieser noch durch flüssigkeitsdurchlässige Abdeckungen getrennt sein. Das Bauteil oder die Schicht aus saugfähigem und flüssigkeitstransportfähigen Material kann integrierter Bestandteil des Medizinischen Verbandes sein oder getrennt vorliegen. Das Bauteil oder die Schicht kann dann so ausgeführt sein, dass eine Befestigung an dem medizinischen Verband möglich ist oder dass das Bauteil bzw. die Schicht unmittelbar am Körper und die übrigen Bauteile bzw. Schichten des medizinischen Verbandes getrennt angebracht werden.

Das Bauteil oder die Schicht aus saugfähigem und flüssigkeitstransportfähigem Material kann Bestandteil eines beliebigen medizinischen Verbandes sein, beispielsweise eines Wund- oder Stützverbandes, aber auch einer Bandage oder einer Orthese.

Das Bauteil oder die Schicht aus saugfähigem und flüssigkeitstransportfähigem Material ist mit Einrichtungen versehen, über die eine Flüssigkeit, gegebenenfalls auch in Form eines Gels oder einer Paste in das Bauteil bzw, in die Schicht eingebracht werden kann.

Das Bauteil oder die Schicht aus saugfähigem und flüssigkeitstransportfähigem Material erlaubt es, bei angelegtem Verband, medizinisch wirksame Substanzen und Präparate in flüssiger Form, gegebenenfalls auch als Gel oder Paste auf die Körperoberfläche aufzubringen, die bei herkömmlichen Verbänden nur durch Abnehmen des Verbandes aufgebracht werden können oder gar nicht, sofern eine Entfernung des Verbandes aus therapeutischen Gründen nicht möglich ist. Alternativ oder im Wechsel mit der Flüssigkeitszufuhr kann auch Luft drucklos oder unter Blasdruck an die Körperoberfläche gebracht werden und dort Trocknung und Kühlung bewirken. Statt Luft kann auch Sauerstoff oder ein medizinisch wirksames Gas an die Körperoberfläche unterhalb des Verbandes herangeführt werden.

Das saugfähige und flüssigkeitstransportfähige Material ist vorzugsweise ein offenporiger Weichschaumstoff. Es kann aber beispielsweise auch ein Abstandsgewebe, ein dreidimensionales Gewirke oder Gestricke oder eine Anordnung von porösen Röhrchen sein. Viele dieser Materialien weisen zugleich einen geringen Strömungswiderstand für gasförmige Substanzen auf, so dass sie zugleich für die Zuführung von gasförmigen Substanzen geeignet sind.
In jedem Fall kann das saugfähige und feuchtigkeitstransportfähige Material vorzugsweise mit Strukturen in Form von Röhrchen oder Kanälen versehen sein, die mehrfache Funktion übernehmen können. Sie können die Flüssigkeitstransportfähigkeit der Schicht oder des Bauteils aus saugfähigem und flüssigkeitstransportfähigen Material erhöhen, sie können zugleich Bestandteil einer Einrichtung zur Einbringung von Flüssigkeiten sein, sie können bewirken, dass Flüssigkeiten oder gasförmige Substanzen gezielt an ausgewählte Behandlungsorte herangeführt werden und sie können die Drucksteifigkeit der Schicht oder des Bauteils aus ansonsten üblicherweise weichem Material erhöhen, so dass die Flüssigkeitstransportfähigkeit nicht unter dem Druck des angelegten Verbandes durch Kompression verloren geht.

Das saugfähige und flüssigkeitstransportfähige Material kann körperseitig durch eine flüssigkeitsdurchlässige, hautfreundliche, insbesondere textile Abdeckung und auf der vom Körper abgewandten Seite durch ein flüssigkeitsdichtes , aber vorzugsweise dampfdurchlässiges Material abgedeckt sein.

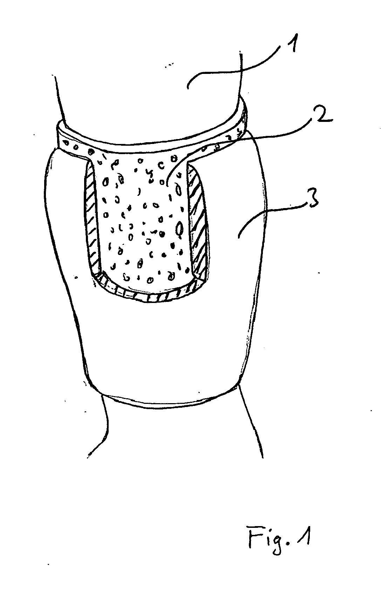

Weitere Merkmale der Erfindung, sowie Ausführungsformen werden an Hand der Figuren 1 bis 12 eräutert.

Figur 1 zeigt die Grundform eines erfinderischen Verbandes. 1 bezeichnet ein beliebiges Körperteil eines menschlichen oder tierischen Körpers. 2 bezeichnet eine dem Körper zugewandte Schicht eines Verbandes oder ein Bauteil aus saugfähigem und flüssigkeitstransportfähigen Material, 3 einen funktionellen Verband oder die vom Körper abgewandte Schicht oder Schichten über der Schicht aus saugfähigem und. flüssigkeitstransportfähigem Material.

Das Bauteil oder die Schicht aus saugfähigem bzw. flüssigkeitstransportfähigem Material kann einfach ein Zuschnitt aus einem offenporigen Weichschaumstoff sein, der auf der Innenseite eines mehrschichtigen Verbandsaufbaus angeordnet ist oder der zuerst an der Körperoberfläche befestigt wird, bevor der eigentliche Körperverband befestigt wird. Das Bauteil oder die Schicht können auch beim Anlegen des Verbandes einfach mit eingebunden werden.

Einrichtungen zum Einbringen einer Flüssigkeit können in diesem Fall in der Form vorliegen, dass der Weichschaumstoff bis zum Rand des Körperverbandes geführt wird und zumindest eine Kante des Schaumstoffes nicht von der Außenschicht oder den Außenschichten des Körperverbandes abgedeckt wird. Über eine Kanüle oder eine Spritze kann die Flüssigkeit zweckmäßig eingebracht werden.

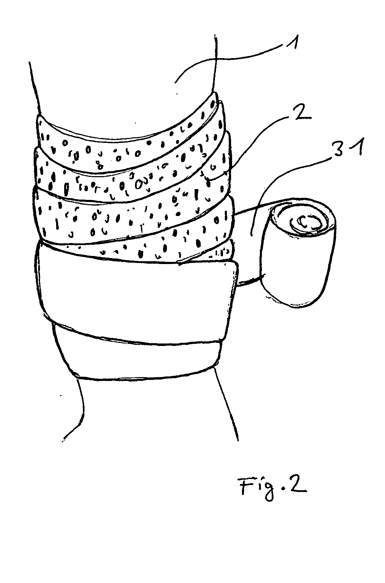

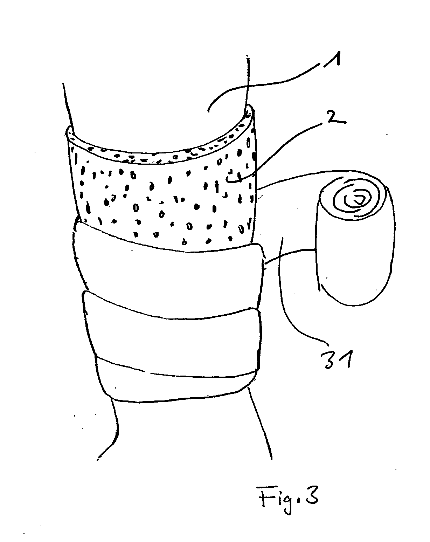

Figur 2 und Figur 3 zeigen Ausführungsformen der Erfindung, bei denen die Schicht 2 aus saugfähigem und flüssigkeitstransportfähigem Material durch eine Binde 31 umwickelt ist. Dabei wird gemäß Figur 2 die Schicht aus saugfähigem bzw. flüssigkeitstransportfähigem
Material 2 einfach durch die inneren Bindenschichten ausgebildet. Gemäß Figur 3 wird ein Bauteil 2 in Form eines Zuschnitts aus saugfähigem bzw. flüssigkeitstransportfähigem
Material auf der Körperoberfläche angebracht und ggfs, fixiert, bevor es durch den Verband 31 umwickelt wird. Das Bauteil 2 kann körperseitig eine flüssigkeitsdurchlässige Abdeckung und auf der vom Körper abgewandten Seite eine flüssigkeitsdichte aber vorzugsweise dampfdurchlässige Abdeckung umfassen. Diese beiden Abdeckungen sind in Figur 3 nicht dargestellt.

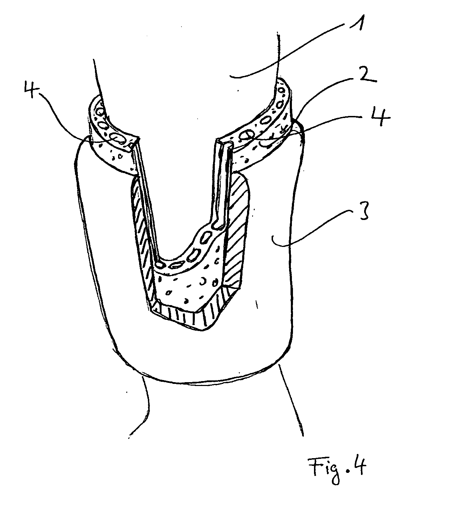

Das saugfähige und flüssigkeitstransportfähige Material kann gemäß Figuren 1 bis 3 beispielsweise ein offenporiger Weichschaum sein. Es kann aber auf Basis eines offenporigen Weichschaums gemäß den Figuren 4 bis 7 durch verschiedene Maßnahmen funktionell optimiert werden.

Gemäß Figur 4 sind Kanäle 4 in den offenporigen Schaumstoff 2 eingearbeitet. Die Kanäle 4 können sich gleichmäßig durch das Material erstrecken oder sie können so angelegt sein, dass sie sich auf Orte hin konzentrieren, an denen eine erhöhte Versorgung mit der Flüssigkeit gewünscht ist.

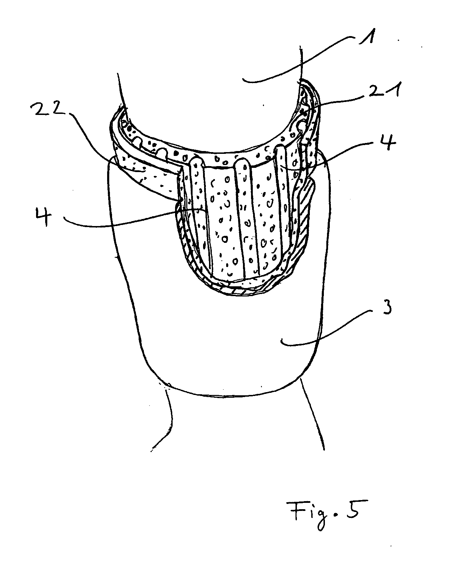

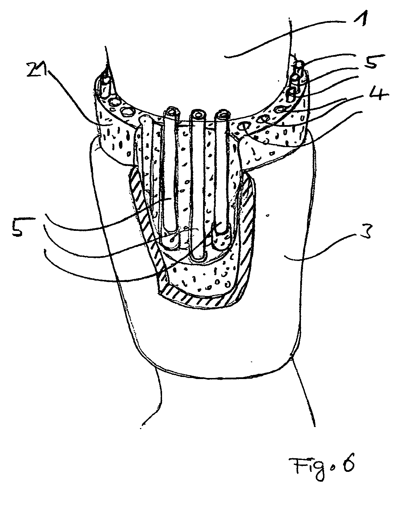

Figur 5 zeigt, wie derartige Kanäle 4 in einfacher Weise ausgeführt sein können. In einen offenporigen Weichschaum 21 werden zunächst Kanäle 4 eingearbeitet, insbesondere geprägt. Diese Kanäle werden anschließend durch eine weitere Schaumstoffschicht 22 abgedeckt, Die beiden Schaumstoffschichten 21, 22 werden beispielsweise durch Verschweißung oder Verklebung verbunden. Die beiden Schichten 21, 22 können aus einem einheitlichen Schaumstoffmaterial bestehen. Es bietet sich aber auch an, die abdeckende Schicht 22 aus einem dichteren Material geringerer Flüssigkeitsleitfähigkeit oder aus vollständig flüssigkeitsundurchlässigem Material auszuführen. Damit wäre bereits eine flüssigkeitsundurchlässige Schicht zur Außenseite des Verbandes hin ausgeführt. Da die Kanalwände durch das saugfähige und flüssigkeitstransportfähige Material selbst gebildet werden, sind sie natürlich auf ganze Länge flüssigkeitsdurchlässig, so dass überall Flüssigkeit aus den Kanälen 4 in das saugfähige und flüssigkeitstransportfähige Material übertreten kann.
Sofern jedoch erwünscht ist, dass Flüssigkeit nur an festgelegten Positionen in das saugfähige und flüssigkeitstransportfähige Material übertritt, so ist dies in einfacher Weise durch die in Figur 6 dargestellte Maßnahme realisierbar. Demnach werden in die Kanäle 4 Röhrchen 5 eingelegt, die nicht flüssigkeitsdurchlässig sind. In der Ausführung nach Figur 6 wurden derartige Röhrchen 5 nur in eine Auswahl der Kanäle 4 eingelegt. Diese Auswahl kann beliebig und nach Bedarf getroffen werden oder es können auch sämtliche Kanäle 4 mit einem derartigen Röhrchen 5 bestückt sein. Ein Flüssigkeitsübertritt in das saugfähige und flüssigkeitstransportfähige Material erfolgt nur dort, wo die Röhrchen 5 enden oder wo sie zum Zweck eines kontrollierten Flüssigkeitsaustritts perforiert sind. Die Röhrchen in den Kanälen 4 gemäß Figur 6 können aus besonders weichem Werkstoff, beispielsweise Siliconkautschuk bestehen, so dass sie den weichen Charakter der Schicht oder des Bauteils aus saugfähigem und flüssigkeitstransportfähigen Material 21 nicht verändern oder sie können aus einem steiferen Kunststoff bestehen, der die Drucksteifigkeit der Schicht oder des Bauteils 21 erhöht, so dass die Schicht oder das Bauteil 21 unter dem Druck des angelegten Verbandes 3 nicht eingedrückt wird.

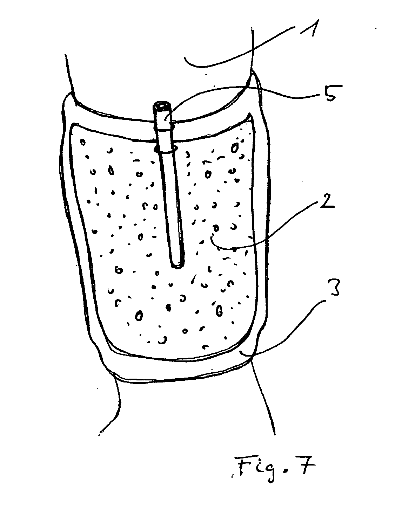

Figur 7 zeigt eine Ausführung, bei der die Schicht oder das Bauteil 2 aus saugfähigem und flüssigkeitstransportfähigen Material vollständig von dem funktionellen Verband 3 überdeckt wird. Um die Schicht oder das Bauteil 2 mit den Kanälen 4 und der Einrichtung zur Zuführung von Flüssigkeiten oder gasförmigen Stoffen in Form von Röhrchen 5 sichtbar zu machen, wurden alle Bauteile transparent dargestellt.
Gemäß Figur 7 können die Röhrchen 5 in der vorstehend beschriebenen Auslegung Bestandteil einer Einrichtung sein, die es erlaubt, Flüssigkeit oder auch gasförmige Substanzen in die Schicht oder das Bauteil 2 aus saugfähigem und flüssigkeitstransportfähigem Material oder aus Material mit hoher Luftfähigkeit für gasförmige Substanzen einzubringen. Dies kann in einfacher Weise dadurch geschehen, dass in den offenen Eingang des Röhrchens oder der Röhrchen 5 beispielsweise durch eine Kanüle oder über eine Spritze Flüssigkeit gegeben wird.
Diese Ausführungsform ist auch zur Einbringung gasförmiger Substanzen geignet, die über ein gasdichtes Röhrchen leicht eingegeben werden können und deren Entweichen durch die allseitige Umhüllung durch den funktionellen Körperverband 3 verhindert oder zumindest behindert wird.

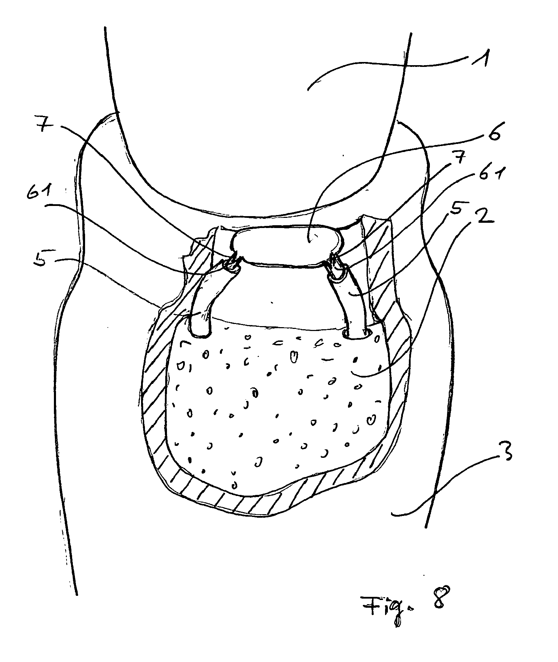

Es ist aber auch möglich, gemäß Figur 8 einen Behälter 6 an den Eingang oder an mehrere Eingänge von Röhrchen 5 anzuschließen. Figur 8 zeigt in einem Ausschnitt des Verbandes beispielshaft ein Reservoir, an das zwei Röhrchen angeschlossen werden können. Natürlich kann der Behälter ebenso nur über einen oder auch über eine größere Anzahl von Röhrchenanschlüssen 61 verfügen.
Figur 8 zeigt eine beispielhafte und sicher gut geeignete Ausführung eines solchen Behälters 6. Dieses besteht beispielweise aus dem selben oder einem ähnlichen weichelastischen Material wie die Röhrchen, also beispielsweise aus Silicon, weichmacherhaltigem PVC oder thermoplastischem Polyurethan. Die Röhrchen 5 werden vorzugsweise einfach auf die passenden Schlauchanschlüsse 61 des Behälters 6 aufgesteckt. Bei Verwendung sehr weichen Materials ist es für eine sichere Steckverbindung zweckmäßig, wenn ein kurzes Adapterröhrchen aus steiferem Werkstoff verwendet wird, das einseitig in den Schlauchanschluß 61 des Behälters 6 und auf der anderen Seite in ein Röhrchen gesteckt wird (Adapterröhrchen nicht dargestellt). Gemäß
Figur 8 verfügt der Behälter 6 für jedes Röhrchen 5 über ein Rückschlag-Flatterventil 7, das nur den Flüssigkeitsaustritt erlaubt. Dieses optionale Ventil kann auch so angeordnet sein, dass es mehrere Schlauchanschlüsse gemeinsam versorgt. Durch Körperbewegung werden Röhrchen 5 und Behälter 6 wechselnd leicht komprimiert und expandiert. Bei diesem Vorgang wird die Flüssigkeit in den Verband gepumpt, das Rückschlagventil 7 verhindert den Rückfluß.
Natürlich sind viele weitere mögliche Ausführungen des Reservoirs die nicht dargestellt sind. Es können auch Behälter mit Absperr- oder Dosierventilen eingesetzt werden. Bei technisch sehr komplexen Einrichtungen zur Zuführung von Flüssigkeiten oder gasförmigen Substanzen wird die Mobilität des Benutzers eingeschränkt werden. Bei einer Einrichtung mit einem Behälter 6 nach Figur 8 wird der Benutzer in seiner Mobilität nicht eingeschränkt werden. Der oder die Behälter sind leicht in alle möglichen Verbände zu integrieren. Dies gilt auch, wenn an verschiedene Röhrchen des Verbandes mehrere Behälter eingesetzt werden.
Ein Behälter nach Figur 8 ist leicht von den Röhrchen 5 zu entfernen, durch eine Kanüle wieder zu befüllen und zum erneuten Gebrauch wieder aufzustecken. Ein derartiger Behälter ist jedoch einfach und billig in der Herstellung und kann, insbesondere aus hygienischen Gründen, auch als Einwegartikel verwendet werden.

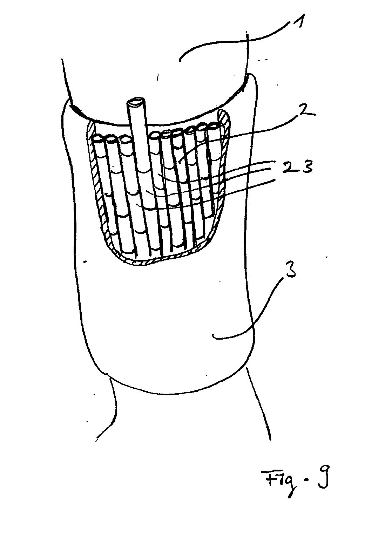

Figur 9 und macht deutlich, dass die Schicht oder das Bauteil 2 aus saugfähigem und flüssigkeitstransportfähigem Material nicht unbedingt als offenporiger Schaumstoff vorliegen muß. Nach dieser Ausführungsform wird die Schicht bzw. das Bauteil 2 durch einen Verbund aus Röhrchen 23 gebildet, beispielsweise aus textilen Röhrchen in Form eines Geflechtes.. Diese Röhrchen 23 können in einer Tasche gefasst sein, deren dem Körper zugewandte Wandung flüssigkeitsdurchlässig und deren vom Körper abgewandte Wandung flüssigkeitsdicht ist.

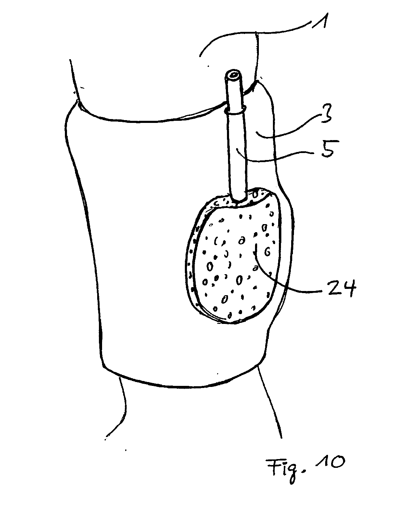

Gemäß allen Darstellungen Figur 1 bis Figur 9 ist die Schicht oder das Bauteil aus saugfähigem und flüssigkeitstransportfähigem Material vollflächig im gesamten Bereich des Verbandes angelegt. Jede der bisher vorgestellten Ausführungsformen kann aber gemäß Figur 10 auch nur in Teilbereichen des Verbandes ausgeführt sein, wenn eine Behandlung mit flüssigen oder gasförmigen Substanzen nur lokal, beispielsweise im Bereich einer Wunde, gewünscht wird. Der Verband 3 ist in Figur 10 transparent dargestellt, um die Lage der Schicht oder des Bauteils 24 aus saugfähigem und flüssigkeitstransportfähigem Material besser darzustellen. Wenn das Bauteil 24 aus einem Material mit geringem Strömungswiderstand besteht, eignet sich diese Lösung auch gut zur Einbringung gasförmiger Substanzen.

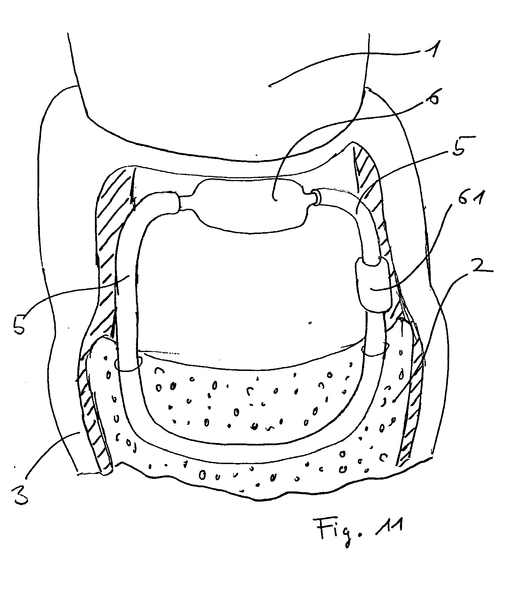

Figur 11 zeigt eine Einrichtung zur Einbringung einer Flüssigkeit in die Schicht oder das Bauteil aus saugfähigem und flüssigkeitstransportfähigem Material 2 in Form eines geschlossenen Kreislaufes. 6 ist ein Behälter, vorzugsweise ein nachfüllbarer Behälter zur Bevorratung einer therapeutisch wirksamen Flüssigkeit. Die Flüssigkeit gelangt über ein Ventil oder eine Pumpe 61 in das Röhrchen oder die Röhrchen 5 , welche die Flüssigkeit in die Schicht oder das Bauteil aus saugfähigem und flüssigkeitstransportfähigem Material 2 leitet. Das Röhrchen 5 ist in diesem Bereich so gestaltet, dass Flüssigkeit in die Schicht oder das Bauteil aus saugfähigem und flüssigkeitstransportfähigem Material 2 abgegeben werden kann, insbesondere perforiert. Flüssigkeit, die durch die Schicht oder das Bauteil aus saugfähigem und flüssigkeitstransportfähigem Material 2 nicht aufgenommen wird, wird durch den geschlossenen Röhrchenkreislauf 5 in den Behälter 6 zurücktransportiert. Die Zirkulation kann durch eine Pumpe oder durch die Komprimierung und Entspannung der flexiblen Röhrchen in Gang aktiviert werden.

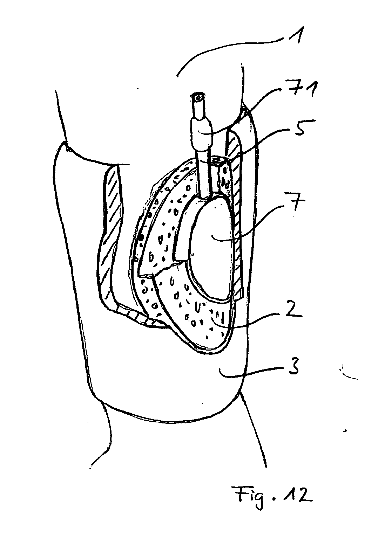

Gemäß Figur 12 ist ein von außen befüllbares Reservoir 7 vorgesehen, das am Ort einer therapeutischen Behandlung eingebaut ist. Das Reservoir 7 kann über eine perforierte oder eine semipermeable Membran Flüssigkeit in die Schicht oder das Bauteil aus saugfähigem und flüssigkeitstransportfähigem Material 2 abgeben. Das Reservoir ist über ein Röhrchen 5 befüllbar. Ein Ventil 71 oder ein Verschluss verhindert Rückströmung der Flüssigkeit.

Es können außerdem in verschiedenen Bereichen des Verbandes mehrere nicht zusammenhängende Bauteile aus saugfähigem und flüssigkeitstransportfähigem Material angeordnet sein, die auch unterschiedlich ausgelegt und mit unterschiedlichen Substanzen versorgt sein können.

Grundsätzlich kann jede der vorgestellten Ausführungsformen in verschiedenartigsten funktionellen Verbänden eingesetzt werden.

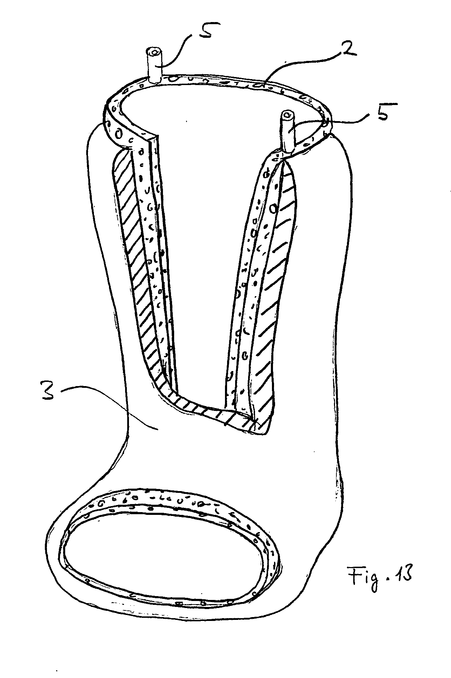

Figur 13 zeigt exemplarisch einen Gipsverband, in den eine vollflächige Schicht oder ein Bauteil 2 aus saugfähigem und flüssigkeitstransportfähigem Material.integriert ist. Der Gipsverband ist zur besseren Übersichtlichkeit ohne das umhüllte Körperteil dargestellt. Gemäß dieser Darstellung umfasst der Verband 3 zwei Schlauchanschlüsse 5 zum Einbringen einer Flüssigkeit oder eines Gases. Die genaue Ausführung der Schicht oder des Bauteils 2 aus saugfähigem und flüssigkeitstransportfähigem Material ist nicht dargestellt. Die konkrete Ausführung kann jeder der Ausführungen gemäß Fig. 4 - 7 oder 9 entsprechen. Es bietet sich bei dieser Ausführungsform an, das Bauteil 2 vor dem Anlegen des Gipsverbandes mit Klebestreifen oder mit Binden am Körperteil zu fixieren

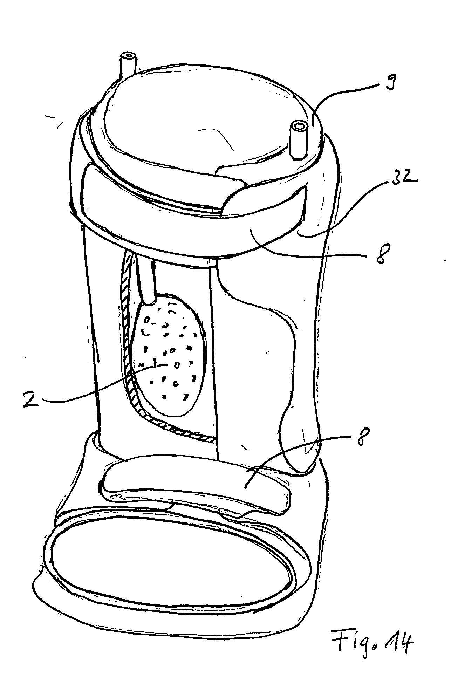

Figur 14 zeigt einen sogenannten Cast 11, d.h. eine fixierende Orthese, die Körpergelenke in vorgegebener Position unbeweglich oder mit einstellbarem Freiheitsgrad fixieren kann. Ein solcher Cast 11 besteht in üblicher Ausführung aus einer Schale 32 aus steifem Kunststoff, die über Verschlusssysteme 8 verfügt. Innerhalb dieser Schale befindet sich ein Kissen 9, das häufig aufblasbar oder evakuierbar ist. Bei geschlossenem Cast 11 passt sich das Kissen 9 dem Körperteil an. Durch Aufblasen oder durch Evakuieren nach der Anpassung wird das Kissen 9 in seiner angepassten Form stabilisiert und hart, so dass der Körperteil ähnlich wie in einem Gipsverband fixiert und stabilisiert ist.
Gemäß der vorliegenden Erfindung werden beispielsweise in beiden Knöchelbereichen Bauteile 2 aus saugfähigem und flüssigkeitstransportfähigem Material integriert. Bei einer derartigen Ausführung bietet es sich an, die erfindungsgemäßen Bauteile durch Klettbefestigungen auf der körperzugewandten Seite derartiger evakuierbarer oder aufblasbarer Polster anzubringen.

## Patentansprüche

1. Körperverband mit mindestens in Teilzonen mehrschichtigem Aufbau, wobei mindestens eine äußere, vom Körper abgewandte Schicht therapeutisch funktionelle Funktionen wie Stabilisierung Lagerung, Fixierung, Korrektur oder Wundabdeckung übernimmt und eine vollflächig oder zumindest in Teilflächen angeordnete innere, der Körperoberfläche zugewandte Schicht aus saugfähigem und flüssigkeitstransportfähigem Material besteht, **dadurch gekennzeichnet, dass** diese der Körperoberfläche zugewandte Schicht zumindest in Teilflächen zur Körperoberfläche hin mit keiner Abdeckung oder mit flüssigkeitsdurchlässigen Abdeckungen versehen ist und dass Einrichtungen vorgesehen sind, um Flüssigkeiten oder pastöse oder gelartige Substanzen ohne Entfernung der äußeren Schicht oder Schichten in die darunter liegenden inneren Schichten aus flüssigkeitstransportfähigem Material einzubringen

2. Bauteil für den Aufbau eines Körperverbandes mit mehrschichtigem Aufbau zur Anordnung auf der Körperoberfläche, das mindestens eine Schicht aus saugfähigem und flüssigkeitstransportfähigem Material umfasst,
**dadurch gekennzeichnet,**
**dass** die Schicht oder die Schichten aus saugfähigem und flüssigkeitstransportfähigem Material zur Körperoberfläche hin zumindest in Teilflächen mit keiner Abdeckung oder mit flüssigkeitsdurchlässigen Abdeckungen versehen ist und dass Einrichtungen vorgesehen sind, um Flüssigkeiten oder pastöse oder gelartige Substanzen in die Schicht oder die Schichten aus saugfähigem und flüssigkeitstransportfähigem Material einzubringen.

3. Körperverband mit mindestens in Teilzonen mehrschichtigem Aufbau, wobei mindestens eine äußere, vom Körper abgewandte Schicht therapeutisch funktionelle Funktionen wie Stabilisierung Lagerung, Fixierung, Korrektur oder Wundabdeckung übernimmt und eine vollflächig oder zumindest in Teilflächen angeordnete innere, der Körperoberfläche zugewandte Schicht einen geringen Strömungswiderstand für gasförmige Substanzen in der Ebene der Körperoberfläche aufweist,
**dadurch gekennzeichnet,**
**dass** die Schicht oder die Schichten mit geringem Strömungswiderstand für gasförmige Substanzen zur Körperoberfläche hin zumindest in Teilflächen mit keiner Abdeckung oder mit Abdeckungen mit hoher Durchlässigkeit für gasförmige Substanzen versehen ist und dass Einrichtungen vorgesehen sind, um gasförmige Substanzen in die Schicht mit geringem Strömungswiderstand hinein- und abzuführen

4. Bauteil für den Aufbau eines Körperverbandes mit mehrschichtigem Aufbau zur Anordnung auf der Körperoberfläche, das mindestens eine Schicht mit geringem Strömungswiderstand für gasförmige Substanzen umfasst.,
**dadurch gekennzeichnet,**
**dass** die Schicht oder die Schichten mit geringem Strömungswiderstand für gasförmige Substanzen zur Körperoberfläche hin zumindest in Teilflächen mit keiner Abdeckung oder mit Abdeckungen mit hoher Durchlässigkeit für gasförmige Substanzen versehen ist und dass Einrichtungen vorgesehen sind, um gasförmige Substanzen .in die Schicht mit geringem Strömungswiderstand hinein- und abzuführen.

5. Körperverband oder Bauteil nach einem oder mehreren der Ansprüche 1 bis 4,
**dadurch gekennzeichnet,**
**dass** das Bauteil gemäß Anspruch 2 oder 4 integrierter Bestandteil eines Körperverbandes mit therapeutischer Funktion wie Stabilisierung, Lagerung, Fixierung, Korrektur oder Wundabdeckung ist.

6. Körperverband oder Bauteil nach einem oder mehreren der Ansprüche 1 bis 4,
**dadurch gekennzeichnet,**
**dass** das Bauteil gemäß Anspruch 2 oder 4 ein unabhängiges Bauteil ist, das in der der Körperoberfläche zugewandten Innenseite eines Körperverbandes mit therapeutischer Funktion wie Stabilisierung, Lagerung, Fixierung, Korrektur oder Wundabdeckung festlegbar ist oder das auf der Körperoberfläche angebracht werden kann bevor ein Körperverband mit therapeutischer Funktion wie Stabilisierung, Lagerung, Fixierung, Korrektur oder Wundabdeckung angebracht wird.

7. Körperverband oder Bauteil nach einem oder mehreren der vorgenannten Ansprüche,
**dadurch gekennzeichnet,**
**dass** die Schicht oder die Schichten aus saugfähigem und flüssigkeitstransportfähigem Material oder die Schicht oder die Schichten mit geringem Strömungswiderstand für gasförmige Substanzen auf der vom Körper abgewandten Seite mit einer im wesentlichen flüssigkeitsdichten und vorzugsweise gasdurchlässigen Abdeckung versehen sind.

8. Körperverband oder Bauteil nach einem oder mehreren der vorgenannten Ansprüche,
**dadurch gekennzeichnet,**
**dass** die Einrichtungen, um Flüssigkeiten oder pastöse oder gelartige Substanzen ohne Entfernung der äußeren Schicht oder Schichten oder Abdeckung in die darunter liegende innere Schicht oder Schichten aus flüssigkeitstransportfähigem Material einzubringen oder um gasförmige Substanzen .in die Schicht mit geringem Strömungswiderstand hinein- und abzuführen Kanäle oder Röhrchen oder Schläuche sind, die an den Seitenkanten oder durch Öffnungen aus dem Körperverband herausgeführt werden.

9. Körperverband oder Bauteil nach einem der vorgenannten Ansprüche,
**dadurch gekennzeichnet,**
**dass** die Schicht oder die Schichten aus saugfähigem und flüssigkeitstransportfähigen Material oder die Schicht oder die Schichten mit geringem Strömungswiderstand für gasförmige Substanzen Einrichtungen enthalten, die geeignet sind, um gasförmige oder flüssige Substanzen in dieser Schicht oder diesen Schichten zu transportieren, zu verteilen oder ausgewählten Punkten zuzuführen.

10. Körperverband oder Bauteil nach Anspruch 9,
**dadurch gekennzeichnet,**
**dass** diese Einrichtungen Kanäle sind, die in die Schicht oder die Schichten aus saugfähigem und flüssigkeitstransportfähigen Material oder die Schicht oder die Schichten mit geringem Strömungswiderstand für gasförmige Substanzen eingearbeitet sind.

11. Körperverband oder Bauteil nach Anspruch 9,
**dadurch gekennzeichnet,**
**dass** diese Einrichtungen eingearbeitete Röhrchen oder Schläuche sind, die in die Schicht oder die Schichten aus saugfähigem und flüssigkeitstransportfähigen Material oder die Schicht oder die Schichten mit geringem Strömungswiderstand für gasförmige Substanzen eingearbeitet sind und die an Orten, an denen die Abgabe von flüssigen oder gasförmigen Substanzen vorgesehen ist, mit Öffnungen oder - Perforationen versehen sind.

12. Körperverband oder Bauteil nach einem oder mehreren der Ansprüche 8 bis 11,
**dadurch gekennzeichnet,**
**dass** Einrichtungen, um Flüssigkeiten oder pastöse oder gelartige Substanzen ohne Entfernung der äußeren Schicht oder Schichten oder Abdeckung in die darunter liegende innere Schicht oder Schichten aus flüssigkeitstransportfähigem Material einzubringen oder um gasförmige Substanzen .in die Schicht mit geringem Strömungswiderstand hinein- und abzuführen und Einrichtungen, um gasförmige oder flüssige Substanzen in dieser Schicht oder diesen Schichten zu transportieren, zu verteilen oder ausgewählten Punkten zuzuführen verbunden sind und von einem Eingang zu einem Ausgang durch den Körperverband oder das Bauteil durchgeleitet werden, derart, dass flüssige oder gasförmige Substanzen durchgeleitet werden können, wobei sie im Bereich der Schicht oder der Schichten aus saugfähigem und flüssigkeitstransportfähigen Material oder in der Schicht mit geringem Strömungswiderstand für gasförmige Substanzen Stoffe abgeben oder aufnehmen können.

13. Körperverband oder Bauteil nach einem oder mehreren der vorgenannten Ansprüche,
**dadurch gekennzeichnet,**
**dass** die Einrichtungen, um Flüssigkeiten oder pastöse oder gelartige Substanzen ohne Entfernung der äußeren Schicht oder Schichten oder Abdeckung in die darunter liegende innere Schicht oder Schichten aus flüssigkeitstransportfähigem Material einzubringen oder um gasförmige Substanzen in die Schicht mit geringem Strömungswiderstand hinein- und abzuführen und Einrichtungen, um gasförmige oder flüssige Substanzen in dieser Schicht oder diesen Schichten zu transportieren, zu verteilen oder ausgewählten Punkten zuzuführen mit Anschlüssen zur Zu- oder Abführung von flüssigen oder gasförmigen Substanzen über Spritzen, Pumpen, Ventilen, Dosierventilen oder Vorratsreservoirs versehen sind.

14. Körperverband oder Bauteil nach einem oder mehreren der vorstehenden Ansprüche,
**dadurch gekennzeichnet,**
**dass** die Schicht oder die Schichten aus saugfähigem und flüssigkeitstransportfähigen Material oder die Schicht oder die Schichten mit geringem Strömungswiderstand für gasförmige Substanzen aus offenporigem Weichschaumstoff oder aus einer dreidimensionalen Textilstruktur wie Abstandsgewebe, einem dreidimensionalen Gestricke oder Gewirke oder einem Verbund aus geflochtenen Röhrchen besteht.

15. Körperverband oder Bauteil nach einem oder mehreren der vorstehenden Ansprüche,
**dadurch gekennzeichnet,**
**dass** der Körperverband oder das Bauteil Stabilisierungselemente enthält zur Vermeidung der Kompression der Schicht oder der Schichten aus saugfähigem und flüssigkeitstransportfähigen Material oder der Schicht oder der Schichten mit geringem Strömungswiderstand für gasförmige Substanzen unter dem Druck des am Körper angepassten Körperverbandes.
